# EUROPÄISCHE PATENTSCHRIFT

(11) **EP 3 711 681 B1**
(45) Veröffentlichungstag und Bekanntmachung des Hinweises auf die Patenterteilung: **10.03.2021**
(21) Anmeldenummer: 20158441.4
(22) Anmeldetag: 20.02.2020
(51) Int. Cl.: A61B 17/86, A61B 17/88, A61B 17/00

(54) **VORSPANNBARES VERRIEGELUNGSSYSTEM**
PRETENSIONABLE LOCKING SYSTEM
SYSTÈME DE VERROUILLAGE POUVANT ÊTRE PRÉCONTRAINT

(30) Priorität: 20.03.2019 DE 102019107198
(43) Veröffentlichungstag der Anmeldung: 23.09.2020
(73) Patentinhaber: Medizin-Mechanik-Nord GmbH, 24111 Kiel (DE)
(72) Erfinder: WITTE, Peter, 24111 Kiel (DE)
(74) Vertreter: Seemann & Partner Patentanwälte mbB

(56) Entgegenhaltungen:
- EP-A2- 0 905 392
- WO-A1-2017/182030
- DE-A1- 19 831 336

## Beschreibung

Die Erfindung betrifft ein vorspannbares Verriegelungssystem zum Einsatz in einem medizintechnischen Instrument, wobei das vorspannbare Verriegelungssystem einen äußeren Verriegelungskörper mit einem Innengewinde und einen inneren Verriegelungskörper mit einem Außengewinde umfasst, wobei in einer Vorspannposition das Innengewinde in einer ersten längsaxialen Richtung an dem Außengewinde anliegt und gegen das Außengewinde vorgespannt ist, wobei in einer Verriegelungsposition der äußere Verriegelungskörper auf dem inneren Verriegelungskörper durch Schrauben des Innengewindes auf das Außengewinde fixiert ist.

Außerdem betrifft die Erfindung ein medizintechnisches Instrument. Vorspannbare Verriegelungssysteme mit einem äußeren Verriegelungskörper und einem inneren Verriegelungskörper kommen in vielen Anwendungen, gerade im medizintechnischen Bereich, zum Einsatz.

Ein Beispiel in der Medizintechnik sind medizinische Instrumente, die einen Innenkörper mit einem ersten Außengewinde und einen Außenkörper mit einem Innengewinde aufweisen. Vor der Verwendung in einer Operation müssen alle Einzelteile eines solchen Instruments desinfiziert werden, wozu das Instrument auseinandergebaut wird. Zur Vorbereitung der Operation wird das Instrument wieder zusammengebaut und der Außenkörper auf dem Innenkörper vorfixiert, jedoch noch nicht festgeschraubt. Das Festschrauben wird erst während der Durchführung der Operation durch den Operateur vorgenommen.

Um den Außenkörper auf dem Innenkörper vorzufixieren, ist es bekannt, auf dem Innenkörper ein zweites Außengewinde vorzusehen, auf das der Außenkörper zum Vorfixieren aufgeschraubt wird. Zwischen dem ersten Außengewinde und dem zweiten Außengewinde des Innenkörpers ist ein Abstand vorgesehen, so dass das Innengewinde des Außenkörpers zwischen dem ersten und dem zweiten Außengewinde vorfixiert ist. Zum endgültigen Fixieren muss der Außenkörper allerdings vom Operateur während der Operation an das erste Außengewinde angedrückt werden, bevor der Außenkörper festgeschraubt werden kann. Dieser zusätzliche Aufwand ist unerwünscht, da der Operateur während der Operation seine volle Aufmerksamkeit der Operation selber schenken sollte.

Beispiele für derartige medizinische Instrumente, insbesondere zur Frakturversorgung, umfassen selbsthaltende Schraubendreher, Gewebeschutzhülsen, Markraumwellenbohrer, Zielgeräte und Schiebehülsen.

Aus WO 2017/182030 A1 ist ein Schraubendreher bekannt, dessen Schraubendreherklinge eine radialkomprimierbare Feder aufweist. Zum Vorfixieren der Hülse auf der Schraubendreherklinge wird das radial nach innen vorstehende Innengewinde der Hülse in längsaxialer Richtung über die Feder geschoben, so dass die Feder radial nach innen gepresst wird. Nachdem das Innengewinde die Feder passiert hat, dehnt sich die Feder wieder radial aus, so dass das Innengewinde der Hülse zwischen der Feder und dem Außengewinde der Schraubendreherklinge festsitzt. In dieser Position ist das Außengewinde in unmittelbarer Nähe zum Innengewinde vorfixiert, so dass zum endgültigen Fixieren der Hülse auf der Schraubendreherklinge die Hülse nicht mehr in Richtung auf das Außengewinde zubewegt werden muss.

EP 0 905 392 A2 zeigt ein Befestigungselement mit einem Gewindeteil und einem davor angeordneten Trägerteil mit einem elastischen Element. Ein Gegengewinde lässt sich mittels des Trägerteils mit dem elastischen Element vor dem Gewindeteil in einen Reibungs- oder Rasteingriff bringen, so dass sich das Gegengewinde in einem vormontierten Zustand vor dem Gewindeteil befindet.

DE 198 31 336 A1 zeigt eine Knochenschraube für den Einsatz bei translaminärer Wirbelverschraubung, wobei ein an einem mit einem Gewinde versehenen Bereich anschließender Bereich unrund nach Art eines Gleichdicks, z.B. trilobular oder mehreckig ausgestaltet ist.

Die Aufgabe der Erfindung besteht darin, ein kostengünstiges, sicheres, in der Anwendung verlässliches und/oder einfach zu bedienendes Verriegelungssystem, insbesondere Einhandverriegelungssystem, zur Verwendung in medizintechnischen Produkten, anzugeben.

Diese Aufgabe wird gelöst durch ein vorspannbares Verriegelungssystem, insbesondere zur Verwendung in medizintechnischen Produkten oder als Teil von medizintechnischen Produkten, umfassend einen als Hülse ausgebildeten äußeren Verriegelungskörper mit einem Innengewinde und einen stabförmigen inneren Verriegelungskörper mit einem Außengewinde, wobei in einer Vorspannposition das Innengewinde in einer ersten längsaxialen Richtung an dem Außengewinde anliegt und gegen das Außengewinde vorgespannt ist, wobei in einer Verriegelungsposition der äußere Verriegelungskörper auf dem inneren Verriegelungskörper durch Schrauben des Innengewindes auf das Außengewinde fixiert ist, wobei das Verriegelungssystem dadurch fortgebildet ist, dass der äußere Verriegelungskörper ein federndes, radial nach innen vorstehendes Halteelement und der innere Verriegelungskörper eine starre, radial nach außen vorstehende Verdickung aufweist, wobei in der Vorspannposition das Halteelement in einer zweiten längsaxialen Richtung, die der ersten längsaxialen Richtung entgegengesetzt ist, an der Verdickung anliegt, wobei die Verdickung rampenförmig ausgebildet ist, wobei ein Steigungswinkel der rampenförmigen Verdickung zwischen 5° und 45° beträgt, wobei das Halteelement ein federnder Haltekranz ist, wobei am äußeren Verriegelungskörper das Halteelement längsaxial beabstandet zum Innengewinde angeordnet ist und am inneren Verriegelungskörper die Verdickung längsaxial beabstandet zum Außengewinde angeordnet ist.

Erfindungsgemäß ist das Außengewinde in der Vorspannposition gegen das Innengewinde in längsaxialer Richtung vorgespannt. Dadurch wird ein besonders einfaches Aufschrauben des äußeren Verriegelungskörpers auf den inneren Verriegelungskörper erreicht. Um das Verriegelungssystem in der Vorspannposition zu halten, sieht das erfindungsgemäße Verriegelungssystem ein federndes, radial nach innen vorstehendes Halteelement des äußeren Verriegelungskörpers und eine starre, radial nach außen vorstehende Verdickung des inneren Verriegelungskörpers vor. Dieses Verriegelungssystem weist vorteilhaft eine geringe Bruchgefahr und eine hohe Lebensdauer auf. Der innere Verriegelungskörper kann durch die Ausbildung der Verdickung als starres Element einer hohen Kraftübertragung standhalten, beispielsweise durch Torsionskräfte. Das erfindungsgemäße Verriegelungssystem ist bruchsicher, übt in der Vorspannposition eine konstante Klemmkraft aus und lässt sich leicht reinigen und desinfizieren. Es kann zudem in vielfältigen Einsatzbereichen eingesetzt und mit geringen Fertigungskosten produziert werden.

Der äußere Verriegelungskörper ist in der Vorspannposition und in der Verriegelungsposition auf den stabförmigen inneren Verriegelungskörper aufgeschoben. Um den äußeren Verriegelungskörper, beispielsweise nach dem Desinfizieren oder Autoklavieren, auf den inneren Verriegelungskörper aufzubringen und in die Vorspannposition zu platzieren, wird der äußere Verriegelungskörper in die erste längsaxiale Richtung auf dem inneren Verriegelungskörper über die Verdickung geschoben, bis das Innengewinde am Außengewinde anliegt und sich das Verriegelungssystem in der Vorspannposition befindet. Da das Innengewinde in der Vorspannposition am Außengewinde anliegt und gegen das Außengewinde vorgespannt ist, lässt sich der äußere Verriegelungskörper einfach auf den inneren Verriegelungskörper aufschrauben. Um den äußeren Verriegelungskörper nach der Operation wieder vom inneren Verriegelungskörper zu entfernen, wird der äußere Verriegelungskörper vom Außengewinde abgeschraubt und in eine zweite längsaxiale Richtung verschoben, so dass das Halteelement über die Verdickung gezogen wird.

Das Halteelement erstreckt sich soweit radial nach innen, dass es beim Verschieben über die Verdickung radial nach außen gebogen wird. Um das Halteelement über die Verdickung zu bewegen, ist daher ein Kraftaufwand erforderlich. Auf diese Weise wird erreicht, dass das Halteelement nicht unbeabsichtigt über die Verdickung rutscht und der äußere Verriegelungskörper in der Vorspannposition sicher auf dem inneren Verriegelungskörper gehalten wird.

In der Verriegelungsposition ist der äußere Verriegelungskörper insbesondere auf dem inneren Verriegelungskörper lösbar und/oder spielfrei fixiert.

Im Rahmen dieser Beschreibung beziehen sich Richtungsangaben wie radial und längsaxial auf das Bezugssystem des stabförmigen inneren Verriegelungskörpers. Das radial nach innen vorstehende Halteelement des äußeren Verriegelungskörpers steht also radial nach innen in Richtung des inneren Verriegelungskörpers vor.

Das erfindungsgemäße Verriegelungssystem ist für eine Vielzahl von medizinischen Anwendungen vorgesehen, beispielsweise medizinische, selbsthaltende Schraubendreher.

Insbesondere ist der äußere Verriegelungskörper als Hülse und/oder der innere Verriegelungskörper stabförmig ausgebildet. Diese Ausbildungsformen sind beispielsweise für eine Anwendung des Verriegelungssystems für einen selbsthaltenden Schraubendreher vorteilhaft.

Das Verriegelungssystem ist vorteilhaft einhändig zu bedienen. Dies erlaubt es einem Operateur, bei der Verwendung eines medizinischen Instruments mit dem erfindungsgemäßen Verriegelungssystem die andere Hand frei zu halten.

Erfindungsgemäß ist die Verdickung rampenförmig ausgebildet, wobei ein Steigungswinkel der rampenförmigen Verdickung zwischen 5° und 45°, insbesondere zwischen 10° und 30°, beträgt.

Als rampenförmige Ausbildung eines Elements wird im Kontext dieser Beschreibung verstanden, dass sich die radiale Ausdehnung des Elements in längsaxialer Richtung zumindest abschnittsweise kontinuierlich verändert. Als Steigungswinkel wird im Kontext dieser Beschreibung der Winkel zwischen einer Rampenfläche der Rampe und der Längsachse des inneren Verriegelungskörpers verstanden.

Vorteilhaft lässt sich das Halteelement durch die rampenförmige Ausbildung der Verdickung mit verhältnismäßig geringem Kraftaufwand über die Verdickung bewegen. Gerade bei flachen Steigungswinkeln wird ein besonders geringer Kraftaufwand erreicht.

Die Verdickung ist bevorzugt in Form einer Doppelrampe mit einer ansteigenden Rampenfläche und einer daran angrenzenden abfallenden Rampenfläche ausgebildet. Hierdurch ist eine leichte und sichere Kopplung mit Instrumentenkomponenten ermöglicht.

Die Verdickung weist somit ein Maximum ihrer radialen Ausdehnung an der Grenzfläche zwischen der ansteigenden Rampenfläche und der abfallenden Rampenfläche auf. Vorteilhaft lässt sich das Halteelement sowohl beim Aufbringen des äußeren Verriegelungskörpers auf den inneren Verriegelungskörper als auch beim Ablösen des äußeren Verriegelungskörpers von dem inneren Verriegelungskörper leicht über die Verdickung bewegen.

Vorzugsweise ist es vorgesehen, dass der innere Verriegelungskörper ein Schraubenschaft oder eine Welle ist.

Die Ausbildung des inneren Verriegelungskörpers als Welle ist beispielsweise bei dem Einsatz des Verriegelungssystems bei einem medizinischen, selbsthaltenden Schraubendreher vorgesehen. In diesem Fall ist die Welle die Schraubendreherklinge.

Das Halteelement ist ein federnder Haltekranz, der insbesondere wenigstens zwei, weiterhin insbesondere wenigstens vier, sich längsaxial erstreckende Laschen aufweist, die an ihren Enden jeweils einen sich radial nach innen erstreckenden Vorsprung aufweisen. In einer beispielhaften Ausführungsform weist der Haltekranz sechs Laschen auf.

Die Ausbildung des Halteelements als Haltekranz stellt vorteilhaft eine günstig herstellbare und stabile Ausführungsform sowie eine garantierte Reinigbarkeit des Halteelements dar. Die Laschen des Haltekranzes sind insbesondere aus einem federnden Material hergestellt. Wird der Haltekranz in längsaxialer Richtung über die Verdickung geschoben, so werden die Vorsprünge und damit die Laschen radial nach außen gedrückt. Nach Überqueren der Verdickung bewegen sich die Vorsprünge durch die Federkraft des Haltekranzes wieder in ihre ursprüngliche radiale Position zurück.

Bevorzugt ist der innere Verriegelungskörper zur Verdrehsicherung in einem an die Verdickung angrenzenden Bereich in einer Mehrkant-, Vielrund-, oder Polygonform ausgebildet. Durch eine derartige Ausbildung des inneren Verrieglungskörpers wird ein sicherer Halt bzw. eine Verdrehsicherung des Halteelements am inneren Verriegelungskörper erreicht, was beispielsweise für Anwendungen in Systemen mit maschinellem Antrieb vorteilhaft ist.

Vorzugsweise ist ein sich längsaxial erstreckender Abschnitt des Halteelements rampenförmig ausgebildet, wobei insbesondere ein Steigungswinkel des rampenförmigen Abschnitts des Halteelements zwischen 10° und 70°, insbesondere zwischen 40° und 50°, beträgt.

Das Vorsehen eines rampenförmigen Abschnitts des Halteelements ist insbesondere dann sinnvoll, wenn die Verdickung eine sich radial erstreckende Haltekante aufweist. Durch den rampenförmigen Abschnitt des Halteelements wird erreicht, dass der äußere Verriegelungskörper aus der Vorspannposition leichter vom inneren Verriegelungskörper ablösbar ist. Insbesondere liegt der rampenförmige Abschnitt des Halteelements in der Vorspannposition an einer Rampenfläche oder einer Haltekante des inneren Verriegelungskörpers an.

Erfindungsgemäß ist das Halteelement am äußeren Verriegelungskörper längsaxial beabstandet zum Innengewinde und die Verdickung am inneren Verriegelungskörper längsaxial beabstandet zum Außengewinde angeordnet.

Mit der Verdickung und dem Halteelement werden somit von den Gewinden getrennte und beabstandete Vorspannmittel bereitgestellt. Das Halteelement und die Verdickung sind in der Vorspannposition räumlich getrennt zum Innengewinde und zum Außengewinde angeordnet. Vorteilhaft wird dadurch ein sicherer Halt des äußeren Verriegelungskörpers auf dem inneren Verriegelungskörper in der Vorspannposition erreicht. Im Unterschied zu Verriegelungssystemen, bei denen das Außengewinde oder das Innengewinde als Vorspannmittel eingesetzt werden, wird vorteilhaft eine Abnutzung des Innengewindes und des Außengewindes vermieden.

Gemäß einer Ausführungsform ist das Außengewinde in der Vorspannposition in längsaxialer Richtung zwischen dem Innengewinde und der Verdickung angeordnet.

In dieser Ausführungsform zieht das Halteelement das Innengewinde auf das Außengewinde. Diese Ausführungsform ist beispielsweise für medizinische Instrumente, wie z.B. Gewebeschutzhülsen, Markraumwellenbohrer, Zielgeräte, selbsthaltende Schraubendreher und Schiebehülsen vorgesehen.

Gemäß einer alternativen Ausführungsform ist das Innengewinde in der Vorspannposition in längsaxialer Richtung zwischen dem Außengewinde und der Verdickung angeordnet.

Gemäß dieser Ausführungsform drückt das Halteelement das Innengewinde auf das Außengewinde.

Vorzugsweise sind der äußere Verriegelungskörper und/oder der innere Verriegelungskörper zumindest teilweise aus Kunststoff hergestellt.

Vorteilhaft ist Kunststoff günstig in der Herstellung. Insbesondere ist der innere Verriegelungskörper vollständig aus Kunststoff hergestellt. Die Herstellung des inneren Verriegelungskörpers aus Kunststoff wird dadurch ermöglicht, dass der innere Verriegelungskörper keine federnden Elemente aufweist, die die Stabilität des inneren Verriegelungskörpers beeinträchtigen würden. Durch die Ausbildung des äußeren Verriegelungskörpers aus Kunststoff lässt sich insbesondere die federnde Eigenschaft des Halteelements gut realisieren und reproduzieren.

Der äußere Verriegelungskörper ist gemäß einer Ausführungsform mehrteilig ausgebildet, wobei ein erstes Bauteil des äußeren Verriegelungskörpers aus einem ersten Material und ein zweites Bauteil des äußeren Verriegelungskörpers aus einem zweiten Material hergestellt ist, das von dem ersten Material verschieden ist.

Beispielsweise ist das erste Bauteil aus Metall und das zweite Bauteil aus Kunststoff hergestellt. Alternativ ist das erste Bauteil aus einem ersten Metall und das zweite Bauteil aus einem zweiten Metall oder das erste Bauteil aus einem ersten Kunststoff und das zweite Bauteil aus einem zweiten Kunststoff hergestellt. Durch geeignete Wahl der unterschiedlichen Materialien lassen sich die Eigenschaften des äußeren Verriegelungskörpers an die jeweiligen Erfordernisse des Verriegelungssystems anpassen, beispielsweise, die Elastizität des Halteelements, die Haltbarkeit des Innengewindes oder die Griffigkeit einer Außenfläche des äußeren Verriegelungskörpers. Hieraus ergeben sich auf den Einsatzbereich anpassbare unterschiedliche Haltekräfte.

Gemäß einer Ausführungsform umfasst der äußere Verriegelungskörper einen Grundkörper, in den das Innengewinde eingebracht ist, und das Halteelement, wobei insbesondere das Halteelement aus Kunststoff hergestellt und in den Grundkörper eingebracht oder an dem Grundkörper befestigt ist.

Der äußere Verriegelungskörper ist in dieser Ausführungsform mehrteilig. Insbesondere ist der Grundkörper aus Metall hergestellt. Die Ausbildung des äußeren Verriegelungskörpers mit einem Halteelement aus Kunststoff und einem Grundkörper aus Metall hat den Vorteil, dass einerseits die elastischen Eigenschaften des Halteelements realisiert und andererseits das Innengewinde des Grundkörpers stabil und langlebig ausgebildet ist.

Zur Herstellung eines äußeren Verriegelungskörpers wird gemäß einer Ausführungsform zunächst ein das Innengewinde aufweisender Grundkörper hergestellt und ein Kunststoff in den Grundkörper, insbesondere mittels eines Spritzgussverfahrens, eingebracht oder auf den Grundkörper aufgetragen. Durch das Einbringen von Kunststoff in den Grundkörper lässt sich ein federndes Halteelement bereitstellen. Der Grundkörper selbst wird insbesondere aus einem harten Material, beispielsweise Metall, hergestellt, um das Innengewinde langlebig und abnutzungsarm auszubilden. Alternativ oder zusätzlich lässt sich mittels des Spritzgussverfahrens eine radiale äußere Schicht aus Kunststoff auf den Grundkörper auftragen. Auf diese Weise lassen sich die haptischen Eigenschaften des äußeren Verriegelungskörpers verbessern.

Vorzugsweise umfasst ein medizintechnisches Produkt, insbesondere ein medizintechnisches Instrument, ein erfindungsgemäßes Verriegelungssystem.

Vorzugsweise weist der innere Verriegelungskörper und/oder der äußere Verriegelungskörper sterilisierbaren Kunststoff auf. Vorzugsweise besteht der innere Verriegelungskörper und/oder der äußere Verriegelungskörper aus sterilisierbarem Kunststoff. Es können auch andere, vorzugsweise flexible Materialien Verwendung finden, die sterilisierbar sind. Hier sei beispielsweise an Titan oder andere Metalle gedacht.

Das erfindungsgemäße Verriegelungssystem wird vorzugsweise als medizintechnisches Adaptionssystem im Bereich der Unfallchirurgie eingesetzt. Es dient hierbei beispielsweise einer eineindeutigen Fixierung von medizinischen Instrumenten und/oder medizinischen Instrumentensystemen, insbesondere bei der Versorgung von Knochenfrakturen an Patienten. Das erfindungsgemäße Verriegelungssystem sichert eine schnelle und einfache sowie sichere Handhabung. Es wird die Operationszeit und ein mögliches Risiko am Patienten deutlich verringert.

Als medizintechnisches Produkt bzw. medizintechnisches Instrument kommt ein medizintechnisches oder medizinisches Instrument in Frage, das einen Innenkörper mit einem ersten Außengewinde und einen Außenkörper mit einem Innengewinde aufweist. Vor der Verwendung in einer Operation werden die Einzelteile eines solchen Instruments desinfiziert, wozu das Instrument auseinander gebaut wird. Zur Vorbereitung der Operation wird das Instrument dann wieder zusammengebaut und der Außenkörper auf den Innenkörper vorfixiert, jedoch noch nicht festgeschraubt. Das Festschrauben wird erst während der Durchführung der Operation durch den Operateur vorgenommen.

Um den Außenkörper auf den Innenkörper vorzufixieren, ist auf dem Innenkörper ein zweites Außengewinde vorgesehen, auf das der Außenkörper zum Vorfixieren aufgeschraubt wird. Zwischen dem ersten Außengewinde und dem zweiten Außengewinde des Innenkörpers ist ein Abstand vorgesehen, so dass das Innengewinde des Außenkörpers zwischen dem ersten und dem zweiten Außengewinde vorfixiert ist. Das erfindungsgemäße Verriegelungssystem sorgt dafür, dass der Außenkörper schon an das erste Außengewinde angedrückt ist, also das Außengewinde und das Innengewinde aneinander kämmen.

Entsprechende medizinische Instrumente, in denen das erfindungsgemäße Verriegelungssystem vorgesehen ist, sind selbsthaltende Schraubendreher, beispielsweise für die distale und/oder proximale Verriegelung von Implantaten, eine Zielgerätfixierung zur Verriegelung von Implantaten, beispielsweise in Form einer Gewebeschutzhülse, eine Welle, die einen Markraumbohrer antreibt, bei der insbesondere das Verriegelungssystem adaptiert werden kann und/oder mehrteilige Instrumente, die vorzugsweise adaptiert und über das Verriegelungssystem fixiert werden können.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
Fig. 1 eine schematisch vereinfachte perspektivische Darstellung eines Verriegelungssystems,
Fig. 2a - 2c eine schematisch vereinfachte Querschnittsansicht eines Verriegelungssystems in einer losen Position, einer Vorspannposition und einer Verriegelungsposition,
Fig. 3 eine schematisch vereinfachte Querschnittsansicht eines Verriegelungssystems am Beispiel eines Schraubenschafts mit einem äußeren Verriegelungskörper und
Fig. 4 eine schematisch vereinfachte Querschnittsdarstellung eines mehrteiligen Verriegelungskörpers mit einem Grundkörper und einem eingebrachten Halteelement.

In den Zeichnungen sind jeweils gleiche oder gleichartige Elemente und/oder Teile mit denselben Bezugsziffern versehen, so dass von einer erneuten Vorstellung jeweils abgesehen wird.

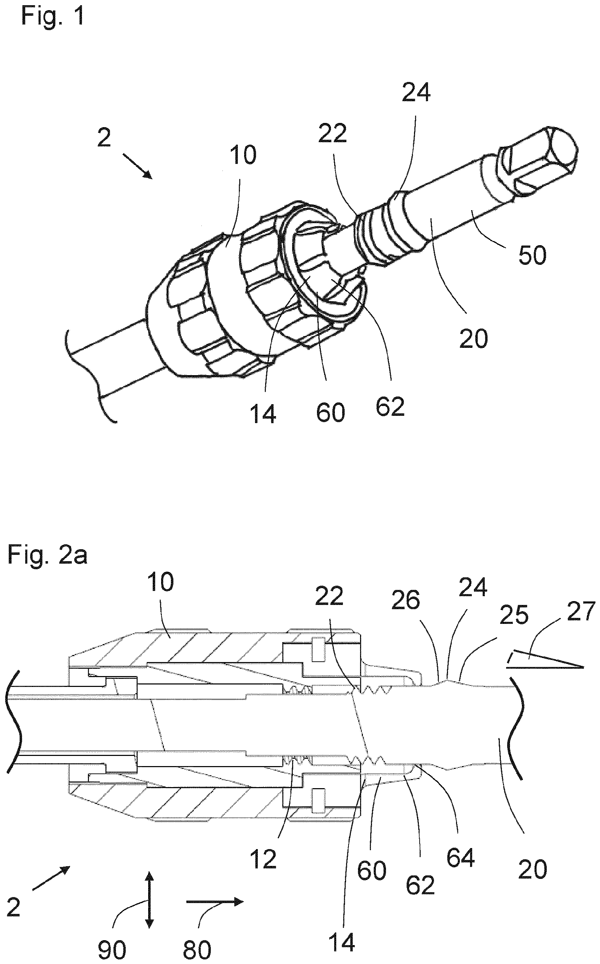

Fig. 1 zeigt schematisch vereinfacht eine perspektivische Ansicht einer beispielhaften Ausführungsform eines Verriegelungssystems 2, wie es z.B. für einen medizinischen, selbsthaltenden Schraubendreher zum Einsatz kommt. Das Verriegelungssystem 2 umfasst einen äußeren Verriegelungskörper 10 in Form einer Hülse, die auf einen inneren stabförmigen Verriegelungskörper 20, in diesem Fall eine Welle 50, geschoben ist. Der innere Verriegelungskörper 20 ist beispielsweise aus Kunststoff oder Metall hergestellt und weist ein Außengewinde 22 und längsaxial versetzt dazu eine Verdickung 24 auf. Der äußere Verriegelungskörper 10 ist beispielsweise aus Kunststoff oder Metall hergestellt und weist ein in Fig. 1 verdecktes Innengewinde und ein Halteelement 14 auf. In der in Fig. 1 dargestellten Ausführungsform handelt es sich bei dem Halteelement 14 um einen Haltekranz 60 mit federnden Laschen 62, von denen in Fig. 1 aus Gründen der Übersichtlichkeit nur eine mit einem Bezugszeichen versehen ist.

Die Funktionsweise der in Fig. 1 gezeigten beispielhaften Ausführungsform des Verriegelungssystems 2 ist in den Fig. 2a bis 2c dargestellt. Wie in Fig. 2a gezeigt, weist der äußere Verriegelungskörper 10 ein Innengewinde 12 auf. Die Verdickung 24 hat die Form einer Doppelrampe mit einer ansteigenden Rampenfläche 25 und einer abfallenden Rampenfläche 26. Der Steigungswinkel 27 der ansteigenden Rampenfläche 25 ist in der in Fig. 2a gezeigten beispielhaft Ausführungsform etwa 15° und der Steigungswinkel der abfallenden Rampenfläche 26 etwa 25°. Die Laschen 62 des Haltekranzes 60 weisen jeweils sich in radialer Richtung 90 erstreckende Vorsprünge 64 auf. Die Vorsprünge 64 erstrecken sich in einer entspannten Position der Laschen 62 in radialer Richtung 90 soweit nach innen, dass sie nicht über die Verdickung 24 geschoben werden können, ohne die Laschen 62 radial nach außen zu verbiegen. In der entspannten Position sind die Vorsprünge 64 jedoch nicht soweit nach innen gebogen, dass sie beim Schieben über das Außengewinde 22 mit dem Außengewinde 22 in Kontakt kommen.

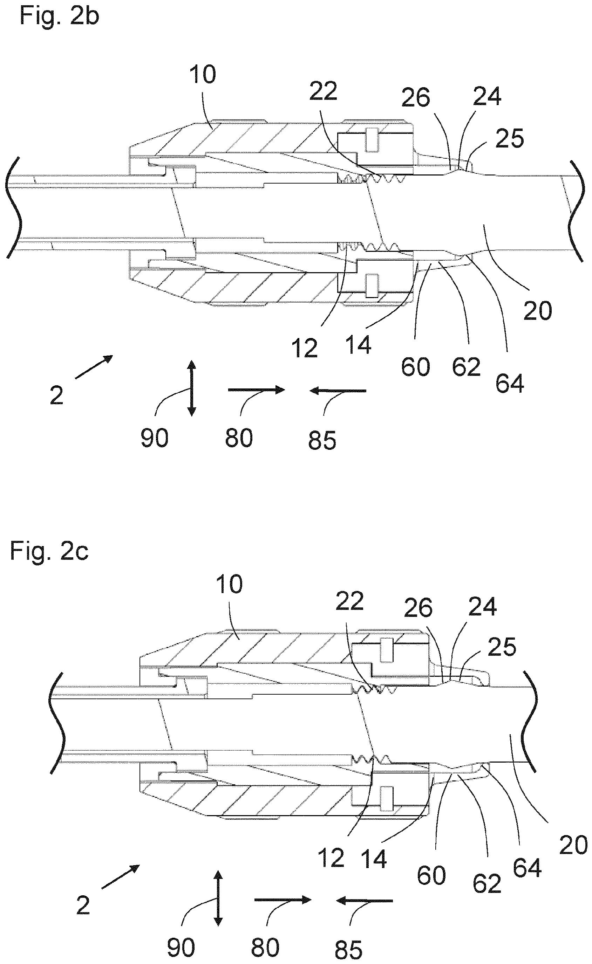

In der in Fig. 2a gezeigten Position ist der äußere Verriegelungskörper 10 lose auf den inneren Verriegelungskörper 20 aufgeschoben. Um den äußeren Verriegelungskörper 10 von dieser Position in eine Vorspannposition zu bewegen, wird der äußere Verriegelungskörper 10 in die erste Längsrichtung 80 geschoben, bis er die maximale radiale Ausdehnung der Verdickung 24 überquert hat und das Innengewinde 12 am Außengewinde 22 in der ersten längsaxialen Richtung 80 anliegt, wie in Fig. 2b gezeigt. Dazu müssen die Vorsprünge 64 der Laschen 62 in radialer Richtung 90 nach außen gebogen werden, wofür ein Kraftaufwand erforderlich ist. In der in Fig. 2b gezeigten Vorspannposition liegen die Vorsprünge 64 an der ansteigenden Rampenfläche 25 der Verdickung 24 an, und zwar in einer zweiten längsaxialen Richtung 85, die der ersten längsaxialen Richtung 80 entgegengesetzt ist, und der radialen Richtung 90. Die Vorsprünge 64 sind in der in Fig. 2b gezeigten Vorspannposition nach außen gebogen, so dass sie eine Federkraft ausüben, die das Innengewinde 12 in der ersten längsaxialen Richtung 80 gegen das Außengewinde 22 drückt.

Aus der Vorspannposition heraus kann der äußere Verriegelungskörper 10 durch eine Rotation des äußeren Verriegelungskörpers 10 auf dem inneren Verriegelungskörper 20 festgeschraubt werden, bis er die in Fig. 2c gezeigte Verriegelungsposition erreicht hat. Da das Innengewinde 12 in der Vorspannposition gegen das Außengewinde 22 vorgespannt ist, ist hierzu lediglich ein Drehen des äußeren Verriegelungskörpers 10 notwendig, ein manuelles Anpressen des Innengewindes 12 an das Außengewinde 22 entfällt.

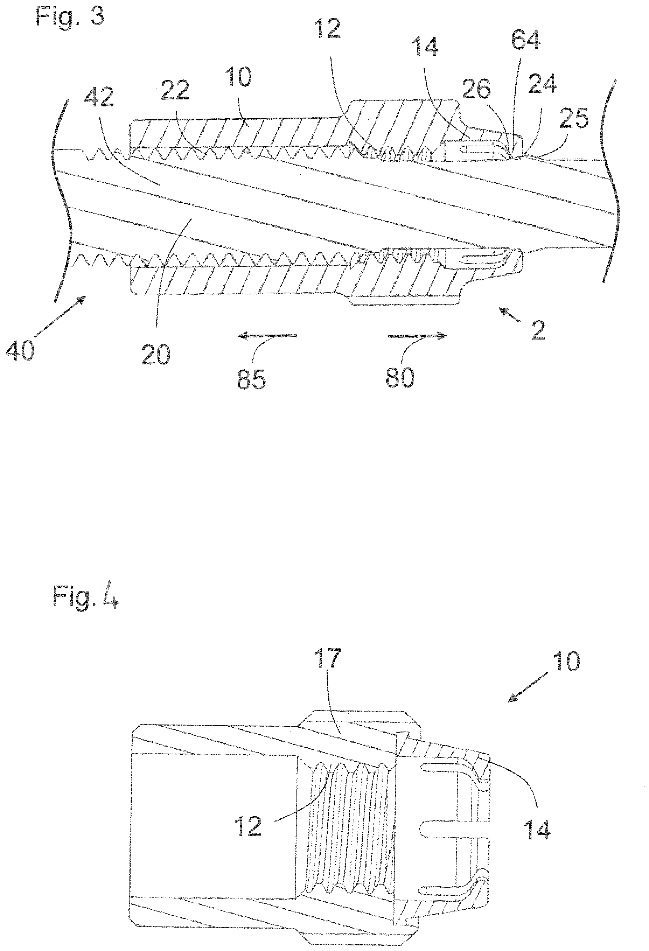

In Fig. 3 ist eine schematisch vereinfachte Querschnittsdarstellung des vorderen Bereichs einer weiteren Ausführungsform eines Verriegelungssystems 2 gezeigt. Mittels diesem Verriegelungssystem 2 wird ein äußerer Verriegelungskörper 10 auf einem inneren Verriegelungskörper 20 in Gestalt eines Schraubenschafts 42 einer Schraube 40 in einer Vorspannposition vorfixiert. Der Schraubenschaft 42 weist ein Außengewinde 22 auf, auf den das Innengewinde 12 des äußeren Verriegelungskörpers 10 geschraubt werden kann. Mit dem Verriegelungssystem 2 ist es möglich, den Verriegelungskörper 10 auf der Schraube 40, bei der es sich insbesondere um eine medizinische Schraube handelt, vorgespannt zu fixieren, so dass der Verriegelungskörper 10 leicht auf das Außengewinde 22 aufgeschraubt werden kann.

Weiterhin ist in Fig. 3 das Innengewinde 12 des Verriegelungskörpers 10 sowie die Verdickung 24 des Schraubenschafts 42 und der Vorsprung 64 des Halteelements 14 zu erkennen. In Fig. 3 befindet sich das Verriegelungssystem 2 in der Vorspannposition. Anders als in Fig. 2b liegt der Vorsprung 64 in der Vorspannposition jedoch nicht an der ansteigenden Rampenfläche 25, sondern an der abfallenden Rampenfläche 26 an. Das Innengewinde 12 ist in dieser Ausführungsform zwischen dem Außengewinde 22 und der Verdickung 24 angeordnet. Dadurch wird das Innengewinde 12 gegen das Außengewinde 22 gedrückt, und nicht wie in Fig. 2b gegen das Außengewinde 22 gezogen.

Fig. 4 zeigt einen äußeren Verriegelungskörper 10, der mehrteilig ausgestaltet ist. Er umfasst das Halteelement 14 und einen Grundkörper 17, der das Innengewinde 12 aufweist. Beispielsweise besteht der Grundkörper 17 aus Metall und das Halteelement 14 aus Kunststoff. Dies hat den Vorteil, dass das Innengewinde 12 durch die Herstellung aus Metall stabil und langlebig ist und gleichzeitig das Halteelement 14 die elastischen Eigenschaften eines Kunststoffes ausnutzt. Zur Herstellung des in Fig. 4 gezeigten äußeren Verriegelungskörpers 10 wird beispielsweise ein Spritzgussverfahren angewendet, d.h. es wird zunächst der Grundkörper 17 hergestellt und anschließend das Halteelement 14 in den Grundkörper 17 eingespritzt.

Die in Fig. 4 gezeigte Ausführungsform des äußeren Verriegelungskörpers 10 ist für eine Vielzahl von medizinischen Anwendungen vorgesehen, beispielsweise medizinische, selbsthaltende Schraubendreher und medizinische Schrauben mit selbsthaltenden Muttern.

### Bezugszeichenliste

- 2: Verriegelungssystem
- 10: äußerer Verriegelungskörper
- 12: Innengewinde
- 14: Halteelement
- 17: Grundkörper
- 20: innerer Verriegelungskörper
- 22: Außengewinde
- 24: Verdickung
- 25: ansteigende Rampenfläche
- 26: abfallende Rampenfläche
- 27: Steigungswinkel
- 40: Schraube
- 42: Schraubenschaft
- 50: Welle
- 60: Haltekranz
- 62: Lasche
- 64: Vorsprung
- 74: innere Mantelfläche
- 80: erste längsaxiale Richtung
- 85: zweite längsaxiale Richtung
- 90: radiale Richtung

## Patentansprüche

1. Vorspannbares Verriegelungssystem (2) zum Einsatz in einem medizintechnischen Instrument, wobei das Verriegelungssystem (2) einen äußeren Verriegelungskörper (10) mit einem Innengewinde (12) und einen inneren Verriegelungskörper (20) mit einem Außengewinde (22) umfasst, wobei in einer Vorspannposition das Innengewinde (12) in einer ersten längsaxialen Richtung (80) an dem Außengewinde (22) anliegt und gegen das Außengewinde (22) vorgespannt ist, wobei in einer Verriegelungsposition der äußere Verriegelungskörper (10) auf dem inneren Verriegelungskörper (20) durch Schrauben des Innengewindes (12) auf das Außengewinde (22) fixiert ist, wobei der äußere Verriegelungskörper (10) ein federndes radial nach innen vorstehendes Halteelement (14) und der innere Verriegelungskörper (20) eine starre, radial nach außen vorstehende Verdickung (24) aufweist, wobei in der Vorspannposition das Halteelement (14) in einer zweiten längsaxialen Richtung (85), die der ersten längsaxialen Richtung (80) entgegengesetzt ist, an der Verdickung (24) anliegt, wobei die Verdickung (24) rampenförmig ausgebildet ist, wobei ein Steigungswinkel (27) der rampenförmigen Verdickung (24) zwischen 5° und 45° beträgt, wobei das Halteelement (14) ein federnder Haltekranz (60) ist, wobei am äußeren Verriegelungskörper (10) das Halteelement (14) längsaxial beabstandet zum Innengewinde (12) angeordnet ist und am inneren Verriegelungskörper (20) die Verdickung (24) längsaxial beabstandet zum Außengewinde (22) angeordnet ist.

2. Verriegelungssystem (2) nach Anspruch 1, **dadurch gekennzeichnet, dass** der Steigungswinkel (27) der rampenförmigen Verdickung (24) zwischen 10° und 30° beträgt.

3. Verriegelungssystem (2) nach Anspruch 2, **dadurch gekennzeichnet, dass** die Verdickung (24) in Form einer Doppelrampe mit einer ansteigenden Rampenfläche (25) und einer daran angrenzenden abfallenden Rampenfläche (26) ausgebildet ist.

4. Verriegelungssystem (2) nach einem der Ansprüche 1 bis 3, **dadurch gekennzeichnet, dass** der innere Verriegelungskörper (20) ein Schraubenschaft (42) oder eine Welle (50) ist.

5. Verriegelungssystem (2) nach einem der Ansprüche 1 bis 4, **dadurch gekennzeichnet, dass** der federnde Haltekranz (60) wenigstens zwei, weiterhin insbesondere wenigstens vier, sich längsaxial erstreckende Laschen (62) aufweist, die an ihren Enden jeweils einen sich radial nach innen erstreckenden Vorsprung (64) aufweisen.

6. Verriegelungssystem (2) nach einem der Ansprüche 1 bis 5, **dadurch gekennzeichnet, dass** zur Verdrehsicherung der innere Verriegelungskörper (20) in einem an die Verdickung (24) angrenzenden Bereich in einer Mehrkant-, Vielrund-, oder Polygonform ausgebildet ist.

7. Verriegelungssystem (2) nach einem der Ansprüche 1 bis 6, **dadurch gekennzeichnet, dass** ein sich längsaxial erstreckender Abschnitt des Halteelements (14) rampenförmig ausgebildet ist, wobei insbesondere ein Steigungswinkel des rampenförmigen Abschnitts des Halteelements (14) zwischen 10° und 70°, insbesondere zwischen 40° und 50°, beträgt.

8. Verriegelungssystem (2) nach einem der Ansprüche 1 bis 7, **dadurch gekennzeichnet, dass** in der Vorspannposition das Außengewinde (22) in längsaxialer Richtung zwischen dem Innengewinde (12) und der Verdickung (24) angeordnet ist.

9. Verriegelungssystem (2) nach einem der Ansprüche 1 bis 8, **dadurch gekennzeichnet, dass** in der Vorspannposition das Innengewinde (12) in längsaxialer Richtung zwischen dem Außengewinde (22) und der Verdickung (24) angeordnet ist.

10. Verriegelungssystem (2) nach einem der Ansprüche 1 bis 9, **dadurch gekennzeichnet, dass** der äußere Verriegelungskörper (10) und/oder der innere Verriegelungskörper (20) zumindest teilweise aus Kunststoff hergestellt sind.

11. Verriegelungssystem (2) nach einem der Ansprüche 1 bis 10, **dadurch gekennzeichnet, dass** der äußere Verriegelungskörper (10) mehrteilig ausgebildet ist, wobei ein erstes Bauteil des äußeren Verriegelungskörpers (10) aus einem ersten Material und ein zweites Bauteil des äußeren Verriegelungskörpers (10) aus einem zweiten Material hergestellt ist, das von dem ersten Material verschieden ist.

12. Verriegelungssystem (2) nach einem der Ansprüche 1 bis 11, **dadurch gekennzeichnet, dass** der äußere Verriegelungskörper (10) einen Grundkörper (17), in den das Innengewinde (12) eingebracht ist, und das Halteelement (14) umfasst, wobei insbesondere das Halteelement (14) aus Kunststoff hergestellt und in den Grundkörper (17) eingebracht oder an dem Grundkörper (17) befestigt ist.

13. Medizintechnisches Instrument umfassend ein Verriegelungssystem (2) nach einem der Ansprüche 1 bis 12.

## Claims

1. A pretensionable locking system (2) for use in a medical-technical instrument, wherein the locking system (2) comprises an outer locking body (10) with an inner thread (12) and an inner locking body (20) with an outer thread (22), wherein in a pretensioned position, the inner thread (12) lies against the outer thread (22) in a first longitudinal axial direction (80) and is pretensioned against the outer thread (22), wherein in a locked position, the outer locking body (10) is fixed on the inner locking body (20) by screwing the inner thread (12) onto the outer thread (22), wherein the outer locking body (10) has a resilient retaining element (14) that projects radially inward and the inner locking body (20) has a rigid bulge (24) that projects radially outward, wherein in the pretensioned position, the retaining element (14) lies against the bulge (24) in a second longitudinal axial direction (85) that is opposite the first longitudinal axial direction (80), wherein the bulge (24) is configured in the shape of a ramp, wherein a gradient angle (27) of the ramp-shaped bulge (24) is between 5° and 45°, wherein the retaining element (14) is a resilient retaining ring (60), wherein the retaining element (14) on the outer locking body (10) is arranged at a longitudinal axial distance from the inner thread (12), and the bulge (24) on the inner locking body (20) is arranged at a longitudinal axial distance from the outer thread (22).

2. The locking system (2) according to Claim 1, **characterized in that** the gradient angle (27) of the ramp-shaped bulge (24) is between 10° and 30°.

3. The locking system (2) according to Claim 2, **characterized in that** the bulge (24) is configured in the form of a double ramp with a rising ramp surface (25) and a falling ramp surface (26) adjacent thereto.

4. The locking system (2) according to any one of Claims 1 to 3, **characterized in that** the inner locking body (20) is a screw shank (42) or a shaft (50).

5. The locking system (2) according to any one of Claims 1 to 4, **characterized in that** the resilient retaining ring (60) has at least two, more particularly at least four, longitudinally axially extending tabs (62) that each have a projection (64) on their ends that extends radially inward.

6. The locking system (2) according to any one of Claims 1 to 5, **characterized in that**, in order to secure against rotation, the inner locking body (20) is configured in a polyhedral, multicurve or polygonal shape in a region adjacent to the bulge (24).

7. The locking system (2) according to any one of Claims 1 to 6, **characterized in that** a longitudinally axially extending section of the retaining element (14) is configured in the shape of a ramp, wherein in particular a gradient angle of the ramp-shaped section of the retaining element (14) is between 10° and 70°, in particular between 40° and 50°.

8. The locking system (2) according to any one of Claims 1 to 7, **characterized in that** in the pretensioned position, the outer thread (22) is arranged in a longitudinal axial direction between the inner thread (12) and the bulge (24) .

9. The locking system (2) according to any one of Claims 1 to 8, **characterized in that** in the pretensioned position, the inner thread (12) is arranged in a longitudinal axial direction between the outer thread (22) and the bulge (24) .

10. The locking system (2) according to any one of Claims 1 to 9, **characterized in that** the outer locking body (10) and/or the inner locking body (20) are at least partially produced from plastic.

11. The locking system (2) according to any one of Claims 1 to 10, **characterized in that** the outer locking body (10) is configured in multiple parts, wherein a first component of the outer locking body (10) is produced from a first material, and a second component of the outer locking body (10) is produced from a second material that is different from the first material.

12. The locking system (2) according to any one of Claims 1 to 11, **characterized in that** the outer locking body (10) comprises a main body (17), into which the inner thread (12) is introduced, and the retaining element (14), wherein the retaining element (14) in particular is produced from plastic and is introduced into the main body (17) or fastened to the main body (17).

13. A medical-technical instrument comprising a locking system (2) according to any one of Claims 1 to 12.

## Revendications

1. Système de verrouillage précontraint (2) destiné à être utilisé dans un instrument médical, le système de verrouillage (2) comprenant un corps de verrouillage extérieur (10) ayant un filetage intérieur (12) et un corps de verrouillage intérieur (20) ayant un filetage extérieur (22) ; dans une position précontrainte, le filetage intérieur (12) vient en butée contre le filetage extérieur (22) dans une première direction axiale longitudinale (80) et est précontraint contre le filetage extérieur (22) ; dans une position de verrouillage, le corps de verrouillage extérieur (10) est fixé sur le corps de verrouillage intérieur (20) par vissage du filetage intérieur (12) sur le filetage extérieur (22), le corps de verrouillage extérieur (10) ayant un élément de retenue élastique (14) faisant saillie radialement vers l'intérieur et le corps de verrouillage intérieur (20) présentant un renflement rigide (24) faisant saillie radialement vers l'extérieur, l'élément de retenue (14), en position de précontrainte, s'appuyant sur le renflement (24) dans une deuxième direction axiale longitudinale (85) opposée à la première direction axiale longitudinale (80), le renflement (24) ayant la forme d'une rampe, un angle (27) d'inclinaison du renflement (24) en forme de rampe étant compris entre 5° et 45°, l'élément de retenue (14) étant un anneau de retenue élastique (60), l'élément de retenue (14) étant disposé sur le corps de verrouillage extérieur (10) à une distance axiale longitudinale du filetage intérieur (12) et le renflement (24) étant disposé sur le corps de verrouillage intérieur (20) à une distance axiale longitudinale du filetage extérieur (22).

2. Système de verrouillage (2) selon la revendication 1, **caractérisé en ce que** l'angle d'inclinaison (27) du renflement (24) en forme de rampe est compris entre 10° et 30°.

3. Système de verrouillage (2) selon la revendication 2, **caractérisé en ce que** le renflement (24) est conçu sous la forme d'une double rampe avec une surface de rampe ascendante (25) et une surface de rampe descendante (26) adjacente.

4. Système de verrouillage (2) selon l'une quelconque des revendications 1 à 3, **caractérisé en ce que** le corps de verrouillage intérieur (20) est une tige filetée (42) ou un arbre (50).

5. Système de verrouillage (2) selon l'une quelconque des revendications 1 à 4, **caractérisé en ce que** l'anneau de retenue élastique (60) comprend au moins deux, en particulier au moins quatre, pattes (62) s'étendant axialement dans le sens longitudinal, dont chacune comprend à ses extrémités une saillie (64) s'étendant radialement vers l'intérieur.

6. Système de verrouillage (2) selon l'une quelconque des revendications 1 à 5, **caractérisé en ce que**, pour empêcher la rotation, le corps de verrouillage intérieur (20) est conçu selon une forme à plusieurs arêtes, multi-arrondie ou polygonale dans une zone adjacente au renflement (24).

7. Système de verrouillage (2) selon l'une des revendications 1 à 6, **caractérisé en ce qu'**une partie de l'élément de retenue (14) s'étendant axialement dans la direction longitudinale est en forme de rampe, un angle d'inclinaison de la partie en forme de rampe de l'élément de retenue (14) étant notamment compris entre 10° et 70°, en particulier entre 40° et 50°.

8. Système de verrouillage (2) selon l'une quelconque des revendications 1 à 7, **caractérisé en ce que**, dans la position de précontrainte, le filetage extérieur (22) est agencé dans la direction axiale longitudinale entre le filetage intérieur (12) et le renflement (24).

9. Système de verrouillage (2) selon l'une des revendications 1 à 8, **caractérisé en ce que**, dans la position de précontrainte, le filetage intérieur (12) est agencé dans la direction axiale longitudinale entre le filetage extérieur (22) et le renflement (24).

10. Système de verrouillage (2) selon l'une quelconque des revendications 1 à 9, **caractérisé en ce que** le corps de verrouillage extérieur (10) et / ou le corps de verrouillage intérieur (20) est / sont au moins partiellement faits en matière plastique.

11. Système de verrouillage (2) selon l'une quelconque des revendications 1 à 10, **caractérisé en ce que** le corps de verrouillage extérieur (10) est formé en plusieurs parties, un premier composant du corps de verrouillage extérieur (10) est constitué d'un premier matériau et un deuxième composant du corps de verrouillage extérieur (10) est constitué d'un deuxième matériau différent du premier matériau.

12. Système de verrouillage (2) selon l'une des revendications 1 à 11, **caractérisé en ce que** le corps de verrouillage extérieur (10) comprend un corps de base (17), dans lequel est introduit le filetage intérieur (12), et l'élément de retenue (14), l'élément de retenue (14) étant notamment en matière plastique et étant introduit dans le corps de base (17) ou étant fixé sur le corps de base (17).

13. Instrument médical comportant un système de verrouillage (2) selon l'une des revendications 1 à 12.
